(19) Europäisches Patentamt

European Patent Office    (11) Publication number: **0 218 568**

Office européen des brevets    **B1**

(12)    EUROPEAN PATENT SPECIFICATION

(45) Date of publication of patent specification: **18.04.90**    (51) Int. Cl.⁵: **A 61 F 5/48**

(21) Application number: **86850331.9**

(22) Date of filing: **02.10.86**

(54) Protector for beds or mattresses.

(30) Priority: **03.10.85 SE 8504583**

(43) Date of publication of application:
**15.04.87 Bulletin 87/16**

(45) Publication of the grant of the patent:
**18.04.90 Bulletin 90/16**

(84) Designated Contracting States:
**AT BE CH DE FR GB IT LI NL SE**

(56) References cited:
**US-A-3 576 039**
**US-A-3 654 059**
**US-A-4 064 577**
**US-A-4 097 943**
**US-A-4 536 433**

(73) Proprietor: **Mölnlycke AB**
**S-405 03 Göteborg (SE)**

(72) Inventor: **Augustsson, Thomas**
**Akergatan 5A**
**S-44233 Kungälv (SE)**

(74) Representative: **Alfredson, Stig et al**
**H. ALBIHNS PATENTBYRA AB Box 3137**
**S-103 62 Stockholm (SE)**

Courier Press, Leamington Spa, England.

**Description**

The present invention relates to a disposable protector for beds or mattresses, which comprises a liquid-absorbing layer and a liquid-impermeable layer intended to face the bedding, the side of the liquid-impermeable layer facing the bedding being at least partially coated with a material which has a high coefficient of friction. Such a protector is known from the disclosure of US—A—4536433.

In hospitals and other types of medical institutions as well as in domestic care, different kinds of bed-protecting devices are being used to prevent urine from penetrating and damaging mattresses.

However, the most common plastic film material that has been used up to now for this purpose is extremely unpleasant for a bed-ridden patient because of the isolation of heat and moisture caused by the film when placed in direct contact with the patient's body. Bed-protecting devices of this type are therefore quite unfit at least for patients confined to bed for long periods of time. Of course, such bed-protecting devices cannot be placed on top of the linens, which will therefore require unnecessarily frequent laundering.

In Swedish Patent Specification 429 717 there is described a bed-protecting device comprising a bottom textile sheet onto which is adhered a sheet of a material which is resistant to water penetration. The distinguishing feature of this prior art bed-protecting device is that the sheet resisting water penetration consists of a finely woven textile material, and that the liquid-absorbing pad is removably secured to said sheet.

However, this type of bed-protecting device has an extremely complicated construction, and does not afford any satisfactory solution with regard to function, either. In fact, the disposable articles has become customary both in hospitals and domestic care by virtue of the more convenient handling as compared with articles for multiple use, which have to be washed for re-utilization. In other contexts such as the supply of diapers in hospitals, it has also been found that the use of disposable articles instead of re-usable products has had an efficiency-improving and cost-saving effect. Another inconvenience with this bed-protecting device is that the underlying sheet, consisting of a material which is resistant to water penetration, has to be wiped dry when changing the detachable portion.

The bed-protecting device known from the afore-said Swedish patent specification has been suggested as an alternative to the disposable liquid-absorbing protectors commonly used during the past few years and having a liquid-tight plastic bottom layer, a liquid-permeable top layer and an absorbent body disposed therebetween. For practical and economic reasons, these protectors are made comparatively small in size with a maximum extension shorter than the width of a normal bed, and are determined for placement in their entirety on top of the bottom cloth sheet, directly beneath the patient. Due to their detachable placement on the cloth sheet, replacement is simple. The idea is obviously that protectors should be easily replaceable a great number of times without also having to change the bedclothes because of wetting. In fact, such loosely attached protectors do not function to full satisfaction as they will easily slip aside or move out of their predetermined positions, thus often being incapable of fulfilling their purpose.

In another known type of bed-protecting device, the risk of sliding has admittedly been eliminated by providing the plastic layer of the device with extending portions to be inserted under the mattress on either long side of the bed. Although this would per se secure a firm attachment of the device, application and replacement would simultaneously become more laborious and time-consuming in comparison with detached devices. On application, these extending portions must be properly tucked in under the mattress by the nursing staff, and for replacement the devices must first be released and the extending portions of the new device then tucked in.

In still another known type, described in U.S. Patent Specification 4 097 943, adhesive beads are applied to the underside of the bed-protecting device. The adhesive will then have to be covered for allowing storage of the devices before use. To eliminate this drawback, the adhering surfaces have been covered with release paper.

This is no satisfactory solution. To cover the adhering surfaces of bed-protecting devices, large amounts of release paper will be required, which is expensive and complicates manufacture. A further drawback is the extra work required to remove the release paper before using the bed-protecting devices.

Moreover, bed protectors with adhering surfaces have been found to function poorly in practice. Such devices namely tend to adhere so strongly to the bedding that they will not all adjust to the movements of the patient. Resulting strains in the protective material will then easily give rise to ruptures.

An adhesive bed-protecting device is also difficult to handle when being inserted in or removed from the bed as it is likely to adhere elsewhere, or to itself; nor can it be withdrawn from the substructure while the patient is still in bed.

It appears from the aforesaid that there has been no success so far in achieving a bed-protecting device which functions satisfactorily in all respects. With the present invention, however, a bed protector of the kind mentioned in the introduction has been obtained by which the disadvantages associated with previously known bed or mattress protecting devices have been entirely eliminated.

For this purpose, a protector for beds or mattresses made according to the invention and comprising a liquid-absorbing layer and a liquid-impermeable layer intended to face the bedding, the side of the liquid-impermeable layer facing the bedding being at least partially coated with a material which has a high coefficient of friction, is distinguished in that the high friction material is a hot melt adhesive having a coefficient of friction according to ASTM D 1894 which is higher than 3 and preferably exceeds 5, the adhesive strength of this hot melt adhesive according to ASTM B 330 M being less than 0.1 N.

By the application of hot melt as a friction lining on the inventive bed protector, the drawback of the device shifting about in the bed as the patient moves is overcome. Despite this, the protector will not be so firmly affixed to the bedclothes that it cannot still be displaced to a certain degree, the device in this manner being prevented from tearing as a result of too great strains occuring in the material.

The hot melt applied onto the inventive bed protector has the usual favourable properties of conventional hot melt for application in a melted state, whereas the free surface of the hot melt in a cold state quite surprisingly lacks adhesive but instead provides a very high frictional force.

The invention will be described in more detail below with reference to the embodiments shown in the accompanying drawings, wherein

Fig. 1 is a perspective view of a bed with the inventive protector for beds or mattresses positioned thereon;

Fig. 2 is a cross section shown to a larger scale of the inventive bed-protecting device; whereas finally

Fig. 3 is a perspective view shown to a larger scale of an embodiment of the inventive bed protector when packed.

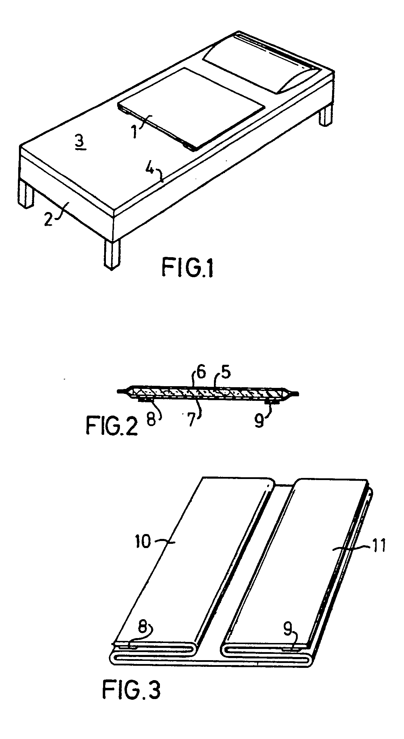

A protector 1 for beds or mattresses made according to the invention is placed, as shown in Fig. 1, upon an ordinary textile sheet 3 right in the center of a mattress or bed 2. In the embodiment shown here, the device is nearly rectangular and so dimensioned that it can be placed in its entirety on top of the mattress or bed. The idea is that it should not be necessary to lift the mattress 4 in order to insert portions of the protector thereunder. The inventive bed protector is prevented from sliding in relation to the textile sheet 3 with the aid of a hot melt applied to its underside as a friction means, the properties of the adhesive naturally being such as to prevent any residues thereof from being left on the bedding after removal of the protector.

The illustrated bed protector consists of an absorbent layer 5 disposed between a liquid-permeable layer 6 of nonwoven textile material and a liquid-impermeable plastic layer 7. On the outside of said plastic layer, hot melt is applied in the form of two elongated beads or strips 8, 9.

For use, the protective device is placed with its portions provided with friction means extending in the longitudinal direction of the bed, which is also the direction where the strains in the device, caused by the movements of the patient, are the greatest.

The inventive bed protectors are suitably supplied in a folded state, as illustrated in Fig. 3. By folding the protectors as shown in this figure, application is facilitated.

In contrast to a bed-protecting device provided with adhesive means, the inventive device is easy to handle. Insertion thereof can be done while the patient is still in bed, and no heavy lifts have to be performed. The patient is rolled to one side of the bed while placing the protector in position and unfolding one half thereof towards the bed, the other half being pleated or folded towards the body of the patient, who is then rolled over to the other side of the bed, whereafter the protector is smoothed out and flattened into full contact with the bedding. The patient can then be rolled over to lie on his back, his buttocks thereby resting on the protector.

For removal of the protector, the process is repeated in the reverse order.

Due to the total lack of adhesivity in the hot melt, applied as a friction means, no release paper will be needed, and the problems associated therewith are thus avoided.

An additional advantage of using hot melt as a friction means is that with known technique, the application of such a glue on an article of the type in question is simple and inexpensive.

With a view of illuminating the properties of the hot melt applied to a bed protector made in accordance with the invention, tests have been carried out the outcome of which is accounted for in the following.

A polyethylene film coated with a hot melt having a friction-generating and non-adhesive effect on bedclothes was tested by the National Swedish Institute for Materials Testing with respect to frictional force and adhesivity at different surface weights in hot melt coatings. A hot melt was used, specially developed for the inventive bed protectors by the company ECOMELT and having the designation ECOMELT H3 EX 479.

Determination of the coefficient of friction for the friction agent was performed according to ASTM D 1894, this testing method involving a sledge of a certain weight being dragged over the friction surface at a certain constant rate, the frictional force then being measured and the coefficient of friction estimated.

The following values of frictional forces and coefficients of friction were obtained at hot melt coatings of different surface weights ($g/m^2$) on polyethylene film:

| Sample | Frictional force (N) | Frictional coefficient (−) |
|---|---|---|
| Zero | 2.5 | 1 |
| 21 g/m² | 7 | 4 |
| 41 g/m² | 10 | 5 |
| 60 g/m² | 13 | 6 |

As is shown, a considerably increased frictional force is obtained when coating the plastic film with the hot melt used here. In order to obtain the desired degree of friction and for manufacturing reasons, a coating of between 40 and 100 g hot melt per square meter of film surface is advantageous.

The adhesivity of the hot melt coating was tested according to ASTM B 3330 M, and the test was performed against glass using four 13 mm wide sample bodies.

The samples were found to adhere to the glass with an adhesive strength less than 0.1 N. This adhesive strength is negligible in practice.

Another example of hot melt with acceptable properties is an admixture of Findley's glue 998—337 and Findley's glue 998—337—01.

The invention is not restricted to the embodiments described above, but a plurality of modifications are conceivable within the scope of the following claim.

Naturally, neither the extension nor the placement of the friction means is limited to what has been suggested in the foregoing. For example, a friction means can be applied solely to the corners of the bed protector, or in a discontinuous pattern across the bottom surface thereof.

The absorbent material in the inventive bed or mattress protector need not be cellulose pulp accommodated between the plastic layer and a liquid-permeable layer, but could just as well consist of a dry-laid or wet-laid fibrous web laminated to a plastic film for providing a bed-protector.

Alternatively, the friction means may be combined with adhering means, for example in the form of pieces of adhesive tape or pressure-sensitive adhesive being applied to the corner of the bed protector. Such restricted adhering areas do not per se provide for the protective device to be securely fixed in the bed but will, in combination with the friction means, give increased security against displacement of the protector in relation to the bedding. The self-adhering areas must of course be made sufficiently small so as not to give rise to the problems associated with such adhesive areas, mentioned by way of introduction.

## Claims

1. A disposable protector for beds or mattresses comprising a liquid-absorbing layer (5) and a liquid-impermeable layer (7) intended to face the bedding, the side of the liquid-impermeable layer (7) facing the bedding being at least partially coated with a material which has a coefficient of friction, said protector being characterized in that the high friction material is a hot melt adhesive (8, 9) having a coefficient of friction according to ASTM D 1894 higher than 3 and preferably exceeding 5, and an adhesive strength less than 0.1 N.

2. A protector for beds or mattresses according to Claim 1, characterized by press-sensitive adhering means such as pieces of adhesive tape or adhesive areas being applied to the corners of the bed protector and having an adhering area which is small in relation to the area of the bed protector.

## Patentansprüche

1. Einweg-Schutzvorrichtung für Betten oder Matratzen mit einer Flüssigkeit-Absorptionsschicht (5) une einer flüssigkeitsundurchlässigen Schicht (7), die dem Bett zugewandt sein soll, wobei die Seite der flüssigkeitsundurchlässigen Schicht (7), die dem Bett zugewandt ist, mindestens teilweise mit einem Werkstoff beschichtet ist, der einen hohen Rebungskoeffizienten aufweist, dadurch gekennzeichnet, daß der Werkstoff hoher Reibung ein heißhärtender Kleber (8, 9) mit einem Reibungskoeffizienten nach ASTM D 1894 (American Society for Testing and Materials) von über 3 ist un vorzugsweise von über 5, und mit einer Klebefestigkeit, die geringer als 0,1 N ist.

2. Schutzvorrichtung für Betten oder Matratzen nach Anspruch 1, dadurch gekennzeichnet, daß druckempfindliche Klebeeinrichtungen, wie beispielsweise Stücke eines Klebebands oder Klebeflächen, an den Ecken der Schutzvorrichtung das Bett aufgebracht sind und einen Haftbereich aufweisen, der im Verhältnis zur Fläche der Schutzvorrichtung für das Bett klein ist.

## Revendications

1. Dispositif de protection jetable pour lit ou matelas, comportant une couche (5) absorbant les liquides

et un couche (7) imperméable aux liquides, prévue pour se trouver du côté de la literie, le côté de la couche (7) impreméable aux liquides faisant face à la literie étant au moins partiellement revêtu d'un matériau qui a un coefficient de friction élevé, ledit dispositif de protection étant caractérisé en ce que le matériau à friction élevée est un adhésif thermoflusible (8, 9) ayant un coefficient de friction selon ASTM D 1894 supérieur à 3 et de préférence supérieur à 5, et une adherence inférieure à 0,1 N.

2. Dispositif de protection pour lit ou pour matelas selon la revendication 1, caractérisé par des moyens d'adhérence sensibles àla pression tels que des moreceaux de bande adhésive ou des surfaces adhésives appliqués sur les coins du dispositif de protection de lit et ayant une surface adhésive qui est petité par rapport à la surface du dispositif de protection de lit.

FIG.1

FIG.2

FIG.3